# EUROPÄISCHE PATENTANMELDUNG

(11) **EP 0 658 371 A1**
(43) Veröffentlichungstag der Anmeldung: **21.06.1995**
(21) Anmeldenummer: 94119295.7
(22) Anmeldetag: 07.12.1994
(51) Int. Cl.: B01D 61/44, C07C 51/02, C07C 227/40

(54) **Verfahren zur Gewinnung von Carbonsäuren aus ihren Salzen**

(30) Priorität: 15.12.1993 DE 4342668
(71) Anmelder: BASF AKTIENGESELLSCHAFT, D-67056 Ludwigshafen (DE)
(72) Erfinder: Voss, Dr. Hartwig, D-67227 Frankenthal (DE)

(57) **Zusammenfassung**

Gewinnung von gesättigten aliphatischen oder cycloaliphatischen Dicarbonsäuren mit 5 bis 10 C-Atomen (I) und mehrwertigen Carbonsäuren der allgemeinen Formel II 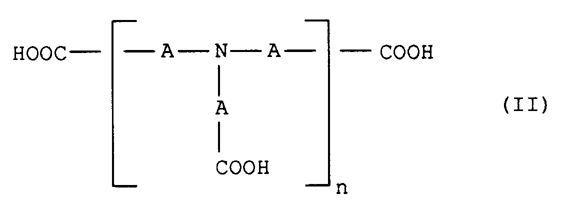
in der die Gruppen A unabhängig voneinander für eine C₁- bis C₄-Alkylengruppe stehen und n eine Zahl von 1 bis 4 bedeutet, durch Elektrodialyse von wäßrigen Lösungen von Salzen oder partiellen Salzen dieser Carbonsäuren, indem man
a) die Elektrodialyse so weit führt, bis ca. 90 % der Carboxylgruppen in freier Form vorliegen,
b) die Carbonsäure aus der resultierenden Lösung außerhalb der Elektrodialysezelle gänzlich oder zum Teil abtrennt und
c) die verbleibende Lösung wieder mit frischem Salz versetzt und in die Elektrodialysestufe zurückführt.

## Beschreibung

Die vorliegende Erfindung betrifft ein Verfahren zur Gewinnung von gesättigten aliphatischen oder cycloaliphatischen Dicarbonsäuren mit 5 bis 10 C-Atomen (I) und mehrwertigen Carbonsäuren der allgemeinen Formel II 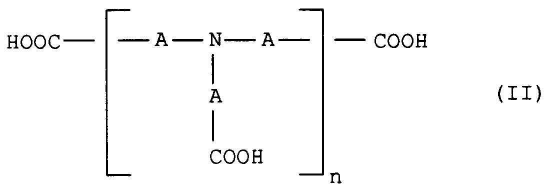
in der die Gruppen A unabhängig voneinander für eine C₁- bis C₄-Alkylengruppe stehen und n eine Zahl von 1 bis 4 bedeutet, durch Elektrodialyse von wäßrigen Lösungen von Salzen oder partiellen Salzen dieser Carbonsäuren.

Bei einer Reihe technischer Prozesse fallen mehrwertige Carbonsäuren in Form wäßriger Lösungen ihrer Salze an.

Beispielsweise erhält man bei der Synthese von Nitrilotriessigsäure aus Formaldehyd, Ammoniak und Cyanwasserstoff und anschließender alkalischer Verseifung des Primärproduktes Tris-(cyanomethyl)-amin ein Nitrilotriessigsäure-Salz.

Auch bei der Hydrolyse von Polyamiden zur Wiedergewinnung der Ausgangsstoffe fallen häufig Dicarbonsäuren als wäßrige Salzlösungen an.

Carbonsäuren in Form ihrer Salze erhält man weiterhin bei fermentativen Syntheseverfahren, wie etwa in der US-A-3 086 928 für Zitronensäure beschrieben.

Es ist allgemein bekannt, zur Gewinnung der zugrundeliegenden mehrwertigen Carbonsäuren derartige Salzlösungen mit einer starken Mineralsäure zu versetzen, wobei die Carbonsäuren meist ausfallen und leicht abgetrennt werden können. Dabei entstehen jedoch auch erhebliche Mengen anorganischer Salze.

Es ist ferner bekannt, mehrwertige Carbonsäuren mittels Elektrodialyse in wäßriger Lösung aus ihren Salzen vollständig freizusetzen und sie aus der resultierenden Lösung beispielsweise durch Kristallisation zu gewinnen. Da die freien Carbonsäuren häufig eine geringere Wasserlöslichkeit aufweisen, als ihre Salze, kristallisieren sie, wenn man sehr konzentrierte Salzlösungen einsetzt, gegen Ende der Elektrodialyse bereits in der Zelle aus, was dazu führen kann, daß die Zelle verstopft und bei weiterem Stromfluß beschädigt wird.

Nach der Lehre der DE-A-2 505 735 läßt sich Zitronensäure aus wäßrigen Lösungen ihrer Alkalimetallsalze gewinnen, indem man sie durch Elektrodialyse, vorzugsweise zu 90 bis 99,9%, freisetzt und die freie Säure aus der so erhaltenen Lösung auskristallisiert. Wegen ihrer guten Wasserlöslichkeit fällt die Zitronensäure in der Elektrodialysestufe nicht aus.

Der vorliegenden Erfindung lag daher ein breit anwendbares und wirtschaftliches Verfahren zur Gewinnung von mehrwertigen Carbonsäuren aus ihren Salzen unter Zuhilfenahme der Elektrodialyse als Aufgabe zugrunde.

Demgemäß wurde ein Verfahren zur Gewinnung von gesättigten aliphatischen oder cycloaliphatischen Dicarbonsäuren mit 5 bis 10 C-Atomen (I) und mehrwertigen Carbonsäuren der allgemeinen Formel II 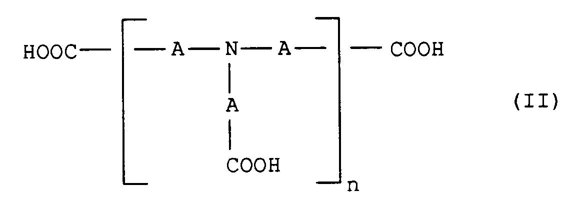
in der die Gruppen A unabhängig voneinander für eine C₁- bis C₄-Alkylengruppe stehen und n eine Zahl von 1 bis 4 bedeutet, durch Elektrodialyse von wäßrigen Lösungen von Salzen oder partiellen Salzen dieser Carbonsäuren gefunden, daß dadurch gekennzeichnet ist, daß man
a) die Elektrodialyse so weit führt, bis ca. 90 % der Carboxylgruppen in freier Form vorliegen,
b) die Carbonsäure aus der resultierenden Lösung außerhalb der Elektrodialysezelle gänzlich oder zum Teil abtrennt und
c) die verbleibende Lösung wieder mit frischem Salz versetzt und in die Elektrodialysestufe zurückführt.

Als gesättigte aliphatische oder cycloaliphatischen Dicarbonsäuren mit 5 bis 10 C-Atomen (I) kommen vorzugsweise cycloaliphatische Vertreter mit 5- bis 7-gliedrigen Ringen wie Cyclopentan-1,2-dicarbonsäure, Cyclohexan-1,2-dicarbonsäure und Cyclohexan-1,4-dicarbonsäure sowie insbesondere aliphatische Vertreter mit unverzweigten Alkylketten wie Glutarsäure, Adipinsäure, Korksäure und Sebacinsäure in Betracht.

Die aliphatischen bzw. cycloaliphatischen Gruppen der Dicarbonsäuren I können durch Sauerstoff und/oder Schwefel unterbrochen sein und Substituenten, z.B. Halogene, Nitro-, Cyano- oder Estergruppen, tragen.

Als mehrwertige Carbonsäuren der allgemeinen Formel II 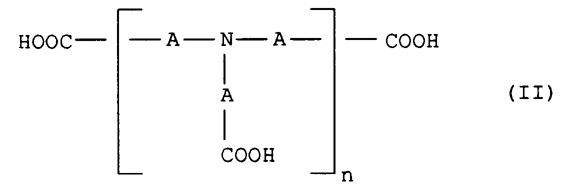
in der die Gruppen A unabhängig voneinander für eine C₁- bis C₄-Alkylengruppe stehen und n eine Zahl von 1 bis 4 bedeutet, sind solche, in denen die Gruppen A unabhängig voneinander für eine C₁- oder C₂-Alkylengruppe stehen und n eine Zahl von 1 bis 3 bedeutet, bevorzugt. Besonders bevorzugt sind Carbonsäuren II der allgemeinen Formel IIa 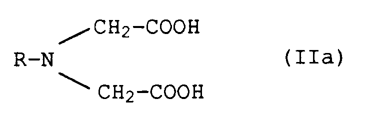
in der R einen der folgenden Reste bedeutet:

-(CH₂)ₘ-COOH

worin m gleich 1 oder 2 ist, oder 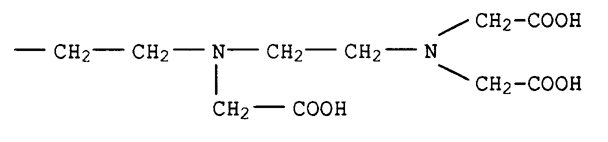
Zur Gewinnung der freien Carbonsäuren I bzw. II geht man beim erfindungsgemäßen Verfahren von den wäßrigen Lösungen ihrer Salze oder partiellen Salze aus, wobei Anmoniumsalze bevorzugt und Alkalimetallsalze besonders bevorzugt sind.

Die Ammoniumsalze enthalten vorzugsweise Ammoniumkationen NX₄⁺, in denen die Substituenten X unabhängig voneinander für Wasserstoff oder C₁- bis C₅-Alkylgruppen stehen wie in Tetraethylammonium-, Tetra-n-butylammonium- oder Triethylmethylammoniumkationen und vor allem in Ammonium- (NH₄⁺) oder Tetramethylammoniumkationen.

Zu den bevorzugten Alkalimetallsalzen gehören Lithium-, Natrium-und Kaliumsalze, von denen Natrium- und Kaliumsalze ganz besonders bevorzugt sind.

Geeignet sind auch gemischte Salze der Carbonsäuren I bzw. II, d.h. solche, die verschiedene Kationen aufweisen.

Erfindungsgemäß führt man die Elektrodialyse so weit, bis ca. 90 % der Carboxylgruppen in freier Form vorliegen.

Hierfür sind solche Elektrodialysevorrichtungen von besonderer Bedeutung, in denen die salzbildenden Kationen über eine Kationenaustauschermembran (KAM) aus der Carbonsäurekammer (CK) in die Kathodenkammer (KK) wandern. Die Kathodenkammer dient dabei als "Basenkammer", weil dort die eintretenden Kationen in der Regel mit den in der Kathodenreaktion gemäß Gleichung (1)

1) 2 H₂O --------> H₂ + 2 OH⁻

gebildeten Hydroxyl-Ionen Basen bilden: 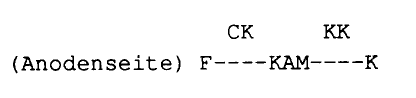
- CK: = Carbonsäurekammer
- KK: = Kathodenkammer ("Basenkammer")
- F: = anodenseitige Begrenzungsfläche der Carbonsäurekammer
- KAM: = Kationenaustauschermembran
- K: = Kathode
Von der anodenseitigen Begrenzungsfläche F der Carbonsäurekammer werden Protonen nachgeliefert, um den Verlust an positiven Ladungsträgern auszugleichen.

Als anodenseitige Begrenzungsfläche F eignet sich die Anode selbst, wo gemäß Gleichung (2) Protonen gebildet werden

2) 2 H₂O ---------> O₂ + 4 H⁺

sowie Sequenzen, welche vorzugsweise aus Anode, Anodenkammer und Kationenaustauschermembran, und vor allem aus Anode, Anodenkammer und bipolarer Ionenaustauschermembran (im folgenden "bipolare Membran" genannt) bestehen und bei denen die Carbonsäuresalzlösung nicht in Kontakt mit der Anode kommt. Man umgeht damit die Gefahr, daß die Carbonsäure die Anode korrodiert und/oder an der Anode oxidiert wird.

Im Falle der für F bevorzugt verwendeten Sequenz aus Anode, Anodenkammer und Kationenaustauschermembran erfolgt der Ladungsausgleich in der Carbonsäurekammer normalerweise durch Protonen, die aus der anodenseitigen Kammer eintreten. Quelle für diese Protonen kann dort beispielsweise die anodische Wasseroxidation gemäß Gleichung (2) sein.

Man kann ferner die Lösung des Carbonsäuresalzes nach Verlassen der Carbonsäurekammer CK durch die anodenseitige Kammer, z.B. die Anodenkammer AK (oder allgemein eine zweite Carbonsäurekammer CK', s.u.), führen, wo eine weitere Abreicherung der salzbildenden Kationen, nunmehr in die Kammer CK, stattfindet: 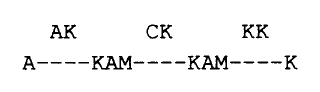
- AK: = Anodenkammer
- A: = Anode
Protonen, die dabei anstelle der salzbildenden Kationen aus der Anodenkammer AK in die Kammer CK übertreten, kommen dem dortigen ersten Elektrodialyseschritt zugute.

Beim erfindungsgemäßen Verfahren besonders bevorzugt sind Elektrodialysezellen, in denen F für eine Sequenz aus Anode, Anodenkammer und bipolarer Membran steht, schematisch: 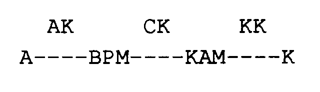
- BPM: = Bipolare Membran
Derartige Zellen sind wegen ihres vergleichsweise einfachen Aufbaus besonders wirtschaftlich.

Bipolare Membranen liefern üblicherweise bei der Elektrodialyse unter Wasserzerlegung anodenseitig Hydroxyl-Ionen und kathodenseitig Protonen.

Daher lassen sich die geeigneten Kationenaustauschermembran-Kammer-Anordnungen (Zellen) auch unter Zwischenschaltung bipolarer Membranen zu Zellstapeln parallel anordnen und betreiben. Die kathodennächste Basenkammer (BK) ist dann zugleich Kathodenkammer.

In einer bevorzugten Ausführungsform des erfindungsgemäßen Verfahrens verwendet man eine Elektrodialysevorrichtung mit einer Membran-Kammer-Anordnung gemäß 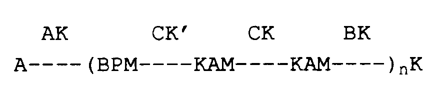
- CK': = zweite Carbonsäurekammer
- BK: = Basenkammer
worin n eine Zahl von 1 bis 500, vorzugsweise von 1 bis 300, bedeutet. Die Carbonsäuresalzlösung durchströmt in der Regel nacheinander die Kammern CK und CK' der jeweiligen Zelle.

In einer besonders bevorzugten Ausführungsform verwendet man eine Elektrodialysevorrichtung mit einer Membran-Kammer-Anordnung gemäß 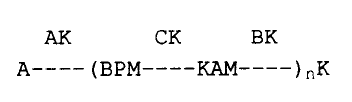
worin n eine Zahl von 1 bis 500, vorzugsweise von 1 bis 300 bedeutet.

Die Anbindung der n-fach wiederkehrenden Membran-Kammer-Anordnungen an die Elektroden kann direkt (s.o.) oder unter Zwischenschaltung weiterer Ionenaustauschermembranen und Kammern erfolgen.

Als Kationenaustauschermembranen eignen sich solche auf Basis von Styrol-Divinylbenzol-Copolymeren, die mit Sulfonsäuregruppen oder anderen anionischen Gruppen funktionalisiert sind oder von perfluorierten Polymeren, welche solche funktionellen Gruppen tragen.

Die geeigneten bipolaren Membranen können vom sog. "single film"-Typ (s. z.B. US-A-4 057 481), oder aus Kationenaustauschermembranen und Anionenaustauschermembranen zusammengesetzt sein (hergestellt z.B. gemäß EP-A-0 193 959 oder J. Electrochem. Soc. 131, Seite 2810 bis 2814 (1984)). Als Anionenaustauschermembranen eignen sind solche auf Basis von Styrol-Divinylbenzol-Copolymeren, die mit quartären Ammoniumgruppen funktionalisiert sind. In der Elektrodialysezelle werden die bipolaren Membranen mit der Anionenaustauscherseite zur Anode hin angeordnet.

Die geeigneten Kationen- und Anionenaustauschermembranen sind handelsüblich.

Die erfindungsgemäß geeigneten Membranen weisen üblicherweise eine Dicke von 0,1 bis 1 mm auf. Der Membranabstand liegt in aller Regel zwischen 0,4 und 3 mm.

Als Anodenmaterialien eignen sich platiniertes Titan und Platin; geeignete Kathodenmaterialien sind Edelstahl und Platin.

Im übrigen ist die Elektrodialyse und besonders der Aufbau der verwendeten Zellen dem Fachmann bekannt (s. z.B. die ältere deutsche Anmeldung P 42 19 758.9), so daß sich weitere Ausführungen hierzu erübrigen.

Die erfindungsgemäße Elektrodialyse führt man so weit, bis ca. 90 % der Carboxylgruppen in freier Form vorliegen.

Insbesondere führt man die Elektrodialyse so weit, bis im Falle einer q-wertigen Carbonsäure, wobei q für einen Wert von 2 bis 6 steht, der prozentuale Anteil der in Salzform vorliegenden Carboxylgruppen in einem Bereich von (100/q)·0,7 bis (100/q)·1,3 liegt, also beispielsweise im Falle einer dreiwertigen Carbonsäure (II) im Bereich von 23,3 bis 43,3%.

Man arbeitet dabei vor allem so, daß es in der Elektrodialysezelle nicht zur Bildung wesentlicher Niederschlagsmengen kommt, setzt jedoch im Hinblick auf eine wirtschaftliche Arbeitsweise in aller Regel möglichst konzentrierte Carbonsäuresalzlösungen ein. Dabei ergibt sich die optimale Konzentration im Einzelfall vor allem aus dem gewünschten Freisetzungsgrad und der Löslichkeit der teilumgesetzten Carbonsäuresalze bei der jeweiligen Elektrodialysetemperatur.

Im allgemeinen sind die teilumgesetzten Carbonsäuresalze in der Wärme besser wasserlöslich als bei Raumtemperatur, weshalb man zugunsten einer höheren Raum-Zeit-Ausbeute eine möglichst hohe Elektrodialysetemperatur wählt, vorzugsweise von 45 bis 80°C.

Der Druck hat keinen erkennbaren Einfluß auf die Elektrodialyse, weshalb man vorzugsweise bei Atmosphärendruck arbeitet.

Die Stromdichte beträgt bei Verwendung bipolarer Membranen in der Regel 20 bis 200 und vorzugsweise 80 bis 120 mA/cm². In den übrigen Fällen beträgt sie vorzugsweise 50 bis 1000 und vor allem 100 bis 400 mA/cm².

Die Strömungsgeschwindigkeit der bei der Elektrodialyse verwendeten Lösungen in den Zellkammern beträgt üblicherweise 0,001 bis 2 m/sec und vor allem 0,01 bis 0,2 m/sec.

Der Grad des elektrodialytischen Austauschs der salzbildenden Kationen gegen Protonen läßt sich in an sich bekannter Weise, z.B. mittels pH- und/oder Leitfähigkeitsmessungen und deren Auswertung anhand von Eichkurven, verfolgen.

Die Elektrodialysestufe (a) kann diskontinuierlich und vorzugsweise, nach den hierfür bekannten Techniken, kontinuierlich durchgeführt werden.

Im übrigen ist die apparative Seite und die Durchführung der Elektrodialyse dem Fachmann bekannt (vgl. z.B. H. Strathmann, "Trennung von molekularen Mischungen mit Hilfe synthetischer Membranen", Steinkopf Verlag, Darmstadt, 1979, Seite 76 bis 86 oder die ältere deutsche Anmeldung P 42 19 758.9).

Von der Lösung, welche aus der Verfahrensstufe (a) resultiert, wird die Carbonsäure außerhalb der Elektrodialysezelle gänzlich oder zum Teil abgetrennt und zwar vorzugsweise mittels Kristallisation oder Extraktion, wobei die Kristallisation besonders bevorzugt ist.

Die Extraktion erfolgt in an sich bekannter Weise, z.B. durch mehrfaches Ausschütteln des Elektrodialyseaustrags mit dem Extraktionsmittel, vorzugsweise mit einem organischen Lösungsmittel, vor allem einem Ether, Sammeln der organischen Phasen und destillatives Entfernen des Extraktionsmittels.

Im Falle der Kristallisation kann man einen Teil des Wassers zunächst vor allem mittels Destillation aus dem Elektrodialyseaustrag entfernen. Dadurch läßt sich in den meisten Fällen die Ausbeute an freier Carbonsäure in der Kristallisationsstufe steigern.

Die Kristallisation wird üblicherweise bei 0 bis 20, vorzugsweise bei 5 bis 15°C vorgenommen.

Das Abkühlen der aus der Verfahrensstufe (a) resultierenden Elektrodialyselösung auf die Endtemperatur der Kristallisation kann in einer und vorzugsweise in mehreren Stufen erfolgen, wobei man darauf achtet, daß keine unerwünschte Mitfällung von Carbonsäuresalzen eintritt.

Der ausgefallene Niederschlag kann diskontinuierlich oder vorzugsweise kontinuierlich, und zwar zweckmäßigerweise mittels Filtration, von der Flüssigphase abgetrennt werden, und er wird in der Regel noch mit Wasser gewaschen.

Kristallisation und Abtrennung der Kristalle können ansonsten wie üblich vorgenommen werden. Hierfür geeignete Vorrichtungen sind z.B. in Ullmanns Encyklopädie der technischen Chemie, 4. Auflage, Verlag Chemie, Weinheim, Band 2, Seite 679 beschrieben.

Bei der erfindungsgemäßen Abtrennung mittels Kristallisation bzw. Extraktion lassen sich die Carbonsäuren I bzw. II mit einem Restgehalt an salzbildenden Kationen von <0,05 Gew.-% gewinnen.

Die nach der Verfahrensstufe (b) verbleibende Lösung, die meist noch geringe Mengen der Carbonsäuren I bzw. II enthält, wird wieder mit frischem Salz versetzt und in die Elektrodialysestufe zurückgeführt.

Das frische Salz kann als solches oder vorzugsweise in Form einer wäßrigen Lösung zugesetzt werden, in der es in der Technik meist anfällt, wobei es dann zweckmäßig ist, der zurückzuführenden Lösung einen Teil des Wassers zu entziehen, um einer fortschreitenden Volumenzunahme der Elektrodialyselösung zu begegnen. Zur Entfernung des Wassers kann man sich allgemein bekannter Verfahren wie der Umkehrosmose oder vorzugsweise der Destillation bedienen.

Die so erhaltene Carbonsäuresalzlösung wird üblicherweise noch außerhalb der Zelle auf die Elektrodialysetemperatur gebracht und dann in die Elektrodialysestufe zurückgeführt.

Das erfindungsgemäße Verfahren kann diskontinuierlich oder nach den hierfür bekannten Techniken vor allem kontinuierlich durchgeführt werden.

Es verläuft wegen der normalerweise höheren Leitfähigkeit der teilumgesetzten Carbonsäuresalze gegenüber der der freien Carbonsäuren, insbesondere bei kontinuierlicher Fahrweise, energetisch günstiger und die meist höhere Wasserlöslichkeit der Salze führt zudem in der Regel zu höheren Raum-Zeit-Ausbeuten, jeweils verglichen mit jenen Verfahren, bei denen die Carbonsäuren mittels Elektrodialyse gänzlich freigesetzt werden.

Die mit dem Verfahren der Erfindung gewonnenen Dicarbonsäuren I eignen sich als Monomere für Polyamide und die mehrwertigen Carbonsäuren II als Komplexbildner in photographischen Entwicklern.

### Beispiele

### Beispiel 1

### Gewinnung von Adipinsäure

### A) Elektrodialyse

### a) Apparatur

Es wurde eine Elektrodialysezelle verwendet, die folgenden schematischen Aufbau hatte: 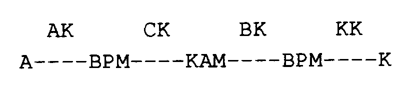
- AK: = Anodenkammer
- CK: = Carbonsäurekammer
- BK: = Basenkammer
- KK: = Kathodenkammer
- A: = Anode
- BPM: = bipolare Membran
- KAM: = Kationenaustauschermembran
- K: = Kathode
Anode und Kathode bestanden aus Platin. Die Kammern CK und BK hatten jeweils einen eigenen, die Kammern AK und KK einen gemeinsamen Außenkreislauf. In allen Außenkreisläufen waren Pumpen, Wärmetauscher und Puffergefäße vorhanden. In den Außenkreisläufen von CK und BK befanden sich zusätzlich pH- und Leitfähigkeitsmeßgeräte.

Als Kationenaustauschermembran diente ein sulfoniertes Styrol-Divinylbenzol-Copolymeres (Selemion^{R} CMV, Fa. Asahi Glass). Die bipolaren Membranen waren gemäß der EP-A-0 193 959 aus einer Kationenaustauschermembran vom Typ Selemion^{R} CMV und einer Anionenaustauschermembran auf Basis eines, durch quartäre Ammoniumgruppen funktionalisierten, Styrol-Divinylbenzol-Copolymeren (Selemion^{R} AMV, Fa. Asahi Glass) hergestellt. Die effektiven Elektroden- und Membranflächen betrugen 37,3 cm² und der Membranabstand 3 mm.

### b) Durchführung

Der Carbonsäurekreis enthielt zu Beginn 220,9 g einer wäßrigen Lösung mit 2,15 mol/kg Adipinsäure-Dinatriumsalz, der Basenkreis 165,2 g Natronlauge der Konzentration 0,12 mol/kg. Im gemeinsamen Spülkreis der Kammern AK und KK befand sich Natronlauge der Konzentration 1 mol/kg.

Die Lösungen wurden bei einer Anfangsstromstärke von 3 A und 50°C 8 Stunden lang umgepumpt. Bis zum Versuchsende fiel die Stromstärke auf 1,7 A und der pH-Wert im Carbonsäurekreis lag zuletzt bei 5,2.

Der Carbonsäurekreis enthielt 179,2 g einer Lösung, die 2,62 mol/kg Protonen und 2,57 mol/kg Natriumionen aufwies, und im Basenkreis lagen 204,9 g Natronlauge der Konzentration 2,44 mol/kg vor.

Die Stromausbeute bezüglich der Protonen betrug 70,3, bezüglich der Natriumionen 73,3%.

### B) Kristallisation

0,5 kg eines Elektrodialyseaustrags gemäß dem vorhergehenden Abschnitt mit 2,62 mol/kg Natriumionen und 2,61 mol/kg Protonen wurden von 50 auf 20°C abgekühlt. Der ausgefallene Niederschlag wurde abfiltriert, mit 270 ml 10°C kaltem Wasser gewaschen und bei 70°C im Vakuum getrocknet. Es wurden 51,07 g Kristallisat mit einem Natriumgehalt von 0,02 Gew.-% und 0,17 Gew.-% Wasser erhalten.

Die Mutterlauge enthielt noch 1,15 mol/kg Protonen und 2,18 mol/kg Natriumionen.

Aus 0,5 kg einer mittels Elektrodialyse aus einer Natriumadipat-Lösung hergestellten Adipinsäure-Lösung, welche ca. 0,55 mol/kg Adipinsäure enthielt (≙ Sättigungskonzentration bei 50°C), konnten unter den gleichen Kristallisationsbedingungen lediglich 27,4 g Dicarbonsäure gewonnen werden.

### C) Extraktion

0,5 kg einer gemäß Abschnitt (b) hergestellten wäßrigen Lösung eines Adipinsäuresalzes, welche 0,994 mol/kg Natriumionen und 0,998 mol/kg Protonen enthielt, wurde dreimal mit jeweils 0,5 kg tert.-Butylmethylether extrahiert. Von den vereinigten organischen Phasen wurde das Extraktionsmittel abdestilliert und es blieben 24,1 g Kristallisat mit einem Natriumgehalt von 0,02 Gew.-% zurück.

### Beispiel 2

### Gewinnung von β-Alanin-N,N-diessigsäure

### A) Elektrodialyse

### a) Apparatur: s. Bsp. 1

### b) Durchführung

Der Carbonsäurekreis enthielt zu Beginn 176,6 g einer wäßrigen Lösung eines partiellen β-Alanin-N,N-diessigsäure-Natriumsalzes mit 0,89 mol/kg Natriumionen und 1,92 mol/kg Protonen, der Basenkreis 159,5 g Natronlauge der Konzentration 2,52 mol/kg und der gemeinsame Spülkreis der Kammern AK und KK Natronlauge der Konzentration 1 mol/kg.

Bei einer Stromstärke von 1,4 A und 50°C wurden der Carbonsäurekammer in 6 Stunden 92,9 g einer wäßrigen technischen Lösung, die 0,886 mol/kg β-Alanin-N,N-diessigsäure-Trinatriumsalz und 0,25 mol/kg Natriumhydroxid enthielt, zugeführt und 79,8 g einer Lösung mit einem Natriumionen-Gehalt von 0,92 mol/kg und einen Protonen-Gehalt von 1,99 mol/kg entnommen.

In der gleichen Zeit wurden dem Basenkammer-Kreis 69,9 g Wasser kontinuierlich zugeführt und 79,7 g Natronlauge der Konzentration 2,42 mol/kg entnommen.

Im Carbonsäurekreis verblieben bei Versuchsende 170,5 g einer Lösung von β-Alanin-N,N-diessigsäure, die 0,92 mol/kg Natriumionen und 1,99 mol/kg Protonen enthielt. Der Basenkreis enthielt 166,1 g einer Natronlauge der Konzentration 2,42 mol/kg.

Die Stromausbeuten betrugen bezüglich der Natriumionen 63 und der Protonen 58 % (Natronlauge in der Ausgangslösung berücksichtigt).

### B) Kristallisation

0,5 kg eines gemäß Abschnitt (b) erhaltenen, 50°C heißen, Elektrodialyseaustrags, welcher 0,96 mol/kg Natriumionen und 1,84 mol/kg Protonen enthielt, wurden auf 0°C abgekühlt. Der ausgefallene Niederschlag wurde abfiltriert, mit 100 ml 0°C kaltem Wasser gewaschen und bei 70°C getrocknet. Es wurden 26,4 g β-Alanin-N,N-diessigsäure mit einem Natriumgehalt von 0,003 Gew.-% erhalten.

Die Mutterlauge enthielt noch 1,13 mol/kg Natriumionen und 1,02 mol/kg Protonen.

Aus 0,5 kg einer mittels Elektrodialyse aus dem Trinatriumsalz hergestellten wäßrigen β-Alanin-N,N-diessigsäure-Lösung, welche ca. 0,13 mol/kg der Tricarbonsäure enthielt (≙ Sättigungskonzentration bei 50°C), konnten unter den gleichen Kristallisationsbedingungen lediglich 13,1 g Carbonsäure gewonnen werden.

## Patentansprüche

1. Verfahren zur Gewinnung von gesättigten aliphatischen oder cycloaliphatischen Dicarbonsäuren mit 5 bis 10 C-Atomen (I) und mehrwertigen Carbonsäuren der allgemeinen Formel II 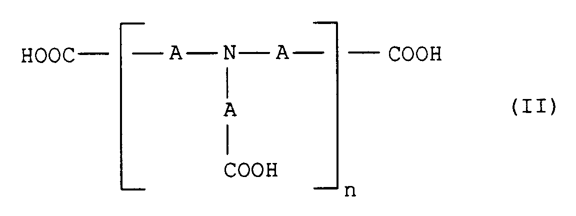 in der die Gruppen A unabhängig voneinander für eine C₁- bis C₄-Alkylengruppe stehen und n eine Zahl von 1 bis 4 bedeutet, durch Elektrodialyse von wäßrigen Lösungen von Salzen oder partiellen Salzen dieser Carbonsäuren, dadurch gekennzeichnet, daß man
a) die Elektrodialyse so weit führt, bis ca. 90 % der Carboxylgruppen in freier Form vorliegen,
b) die Carbonsäure aus der resultierenden Lösung außerhalb der Elektrodialysezelle gänzlich oder zum Teil abtrennt und
c) die verbleibende Lösung wieder mit frischem Salz versetzt und in die Elektrodialysestufe zurückführt.

2. Verfahren nach Anspruch 1, dadurch gekennzeichnet, daß man als Salze oder partielle Salze von Dicarbonsäuren I solche von gesättigten aliphatischen Dicarbonsäuren mit unverzweigten Alkylketten einsetzt.

3. Verfahren nach Anspruch 1, dadurch gekennzeichnet, daß man als Salze oder partielle Salze von mehrwertigen Carbonsäuren II solche der allgemeinen Formel IIa 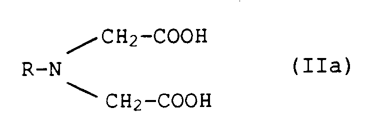 einsetzt, in der R einen der folgenden Reste bedeutet:
-(CH₂)ₘ-COOH
worin m gleich 1 oder 2 ist, oder

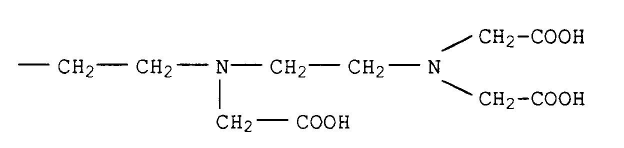

4. Verfahren nach den Ansprüchen 1 bis 3, dadurch gekennzeichnet, daß man von den Natrium- oder Kaliumsalzen der Carbonsäuren ausgeht.

5. Verfahren nach den Ansprüchen 1 bis 4, dadurch gekennzeichnet, daß man in der Verfahrensstufe (a) die Carbonsäure soweit freisetzt, daß im Falle einer q-wertigen Carbonsäure der prozentuale Anteil der in Salzform vorliegenden Carboxylgruppen in einem Bereich von (100/q)·0,7 bis (100/q)·1,3 liegt, wobei q für einen Wert von 2 bis 6 steht.

6. Verfahren nach den Ansprüchen 1 bis 5, dadurch gekennzeichnet, daß man die Elektrodialyse bei 45 bis 80°C durchführt.

7. Verfahren nach den Ansprüchen 1 bis 6, dadurch gekennzeichnet, daß man die Carbonsäure in der Verfahrensstufe (b) durch Kristallisation oder Extraktion abtrennt.

8. Verfahren nach den Ansprüchen 1 bis 7, dadurch gekennzeichnet, daß in der Elektrodialysezelle diejenige Kammer, welcher die Carbonsäure in Form ihres Salzes oder partiellen Salzes zugeführt wird, anodenseitig von einer bipolaren Ionenaustauschermembran und kathodenseitig von einer Kationenaustauschermembran begrenzt ist.

9. Verfahren nach Anspruch 8, dadurch gekennzeichnet, daß die Elektrodialysezelle folgendermaßen aufgebaut ist: Anodenkammer/bipolare Ionenaustauschermembran/Carbonsäurekammer/Kationenaustauschermembran/Basenkammer.

10. Verfahren nach Anspruch 9, dadurch gekennzeichnet, daß in der Elektrodialysevorrichtung bis zu 300 Zellen des Aufbaus: bipolare Ionenaustauschermembran/Carbonsäurekammer/Kationenaustauschermembran/Basenkammer zwischen Anode und Kathode nebeneinander angeordnet sind.
